# EUROPÄISCHE PATENTSCHRIFT

(11) **EP 0 590 386 B1**
(45) Veröffentlichungstag und Bekanntmachung des Hinweises auf die Patenterteilung: **19.11.1997**
(21) Anmeldenummer: 93114553.6
(22) Anmeldetag: 10.09.1993
(51) Int. Cl.: A61F 2/64, A61F 2/66, A61F 2/58

(54) **Gelenk in orthopädischen Prothesen und Orthesen**
Joint for orthopedic prostheses and orthoses
Articulation pour prothèses et orthèses orthopédiques

(30) Priorität: 29.09.1992 DE 4232602
(43) Veröffentlichungstag der Anmeldung: 06.04.1994
(73) Patentinhaber: Otto Bock Orthopädische Industrie Besitz- und Verwaltungs-Kommanditgesellschaft, 37115 Duderstadt (DE)
(72) Erfinder: Krieger, Wilfried, Dipl.-Ing., D-37085 Göttingen (DE)
(74) Vertreter: Gramm, Werner, Prof., Dipl.-Ing.

(56) Entgegenhaltungen:
- EP-A- 0 482 809
- US-A- 4 090 264
- DESIGN ENGINEERING Bd. 232 , Mai 1990 , LONDON GB Seite 51 'Flexible artificial knee joint'

## Beschreibung

Die Erfindung betrifft ein Gelenk oder Schwenkverbindung in orthopädischen Prothesen und Orthesen, mit einem Gelenkoberteil, einem Gelenkunterteil und in polyzentrischer Ausführung mit zusätzlich zwischengeschalteten Lenkern (Gelenkober- und -unterteil sowie Lenker, nachfolgend zusammengefaßt als "Gelenkglieder" bezeichnet) und mit einem Steuerelement, das beidseitig über jeweils einen von einer Gelenkachse beabstandeten Anlenkpunkt wirkungsmäßig an jeweils einem Gelenkglied (nachfolgend "Anlenk-Gelenkglied") derart angreift, daß sich der Abstand zwischen seinen Anlenkpunkten in Abhängigkeit von der Gelenkbewegung oder Verschwenkung verändert.

Dieses Gelenk kann z. B. ein monozentrisches oder polyzentrisches Prothesen-Kniegelenk sein, wobei es nicht auf die Konfiguration der einzelnen Gelenkglieder ankommt.

Im Rahmen der Erfindung kommt es nicht darauf an, ob die Steuerelemente bzw. Steuersysteme auf ein einzelnes Gelenk bzw. eine einzelne Schwenkverbindung oder aber auf mehrere Gelenke bzw. Schwenkverbindungen gleichzeitig einwirken.

Diese Bewegungskontrolle der Gelenke in orthopädischen Prothesen und Orthesen gelingt bei den bekannten Ausführungsformen nur in eingeschränktem Maße. Dies liegt vor allem daran, daß die Bewegungskontrolle in vielen Fällen passiv erfolgt. Die technischen Funktionselemente können in diesen Fällen nicht selbst agieren, z. B. von sich aus Bewegungen generieren, sondern sie beschränken sich in der Regel darauf, passiv auf Bewegungen zu reagieren, indem sie mit bewegungshemmenden und/oder federelastisch puffernden Systemen auf die Bewegungen einwirken. Dies gilt typischerweise für die Gelenke von orthopädischen Beinprothesen. So werden z. B. in bekannten Prothesen-Kniegelenken vorzugsweise bewegungshemmende und/oder federelastisch puffernde Systeme eingesetzt, die entweder grundsätzlich jeder Gelenkbewegung entgegenwirken oder gezielt einzelne Gelenkbewegungen durch Einsatz vorher gespeicherter Energie unterstützen. Ein bekanntes Beispiel für ein federelastisch pufferndes Funktionselement ist der früher in konventionellen sogenannten Knie-Waden-Paßteilen häufig verwendete elastische Kniestreckgurt, der am Ober- und Unterschenkel der Prothese fixiert wird und so das Kniegelenk auf der Vorderseite überbrückt. Er wird mit Einleitung einer Kniebeugung aus der vorgespannten Strecklage zunehmend gespannt. Demzufolge wirkt er der Kniebeugebewegung entgegen und untstützt die Rückkehrbewegung in die Strecklage. Dieses Funktionselement hat allerdings einen wesentlichen Mangel: Das aus der Gurtwirkung resulierende Kniestreckmoment steigt kontinuierlich mit zunehmendem Kniebeugewinkel an. Dadurch ist die unbeschwerte Einnahme der Sitzposition, bei der sich das Prothesen-Kniegelenk in annähernd 90° Beugestellung befindet, ohne zusätzliche technische Maßnahmen eingeschränkt, da der in dieser Körperhaltung weitgehend unbelastete Prothesen-Unterschenkel bestrebt ist, in seine Strecklage zurückzukehren.

In zeitgemäßen orthopädischen Prothesen und Orthesen werden zur Gelenksteuerung Steuerelemente bzw. Steuersysteme eingesetzt, die zwischen einzelnen Gelenkgliedern derart angelenkt sind, daß sich der Abstand zwischen ihren Anlenkpunkten in Abhängigkeit von der Gelenkbewegung bzw. Verschwenkung verändert. Derartige Steuerelemente sind z. B. in Ellbogengelenken, Knöchel-, Knie- und Hüftgelenken orthopädischer Beinprothesen, im Handgelenk- und Ellbogenbereich orthopädischer Armorthesen oder in Knöchel-, Knie- und Hüftgelenken orthopädischer Beinorthesen bekannt.

Am weitesten verbreitet sind Steuerelemente der genannten Art jedoch in monozentrischen und polyzentrischen Kniegelenken orthopädischer Beinprothesen. Daher wird in den nachfolgenden Ausführungen überwiegend auf diesen Einsatzbereich Bezug genommen. Hier dienen die Steuerelemente der Erhöhung der Kniesicherheit der gestreckten Prothese während der Standphase der Gehbewegung, in der die Prothese den Körper unterstützt und/oder der Steuerung der Bewegung des Prothesen-Unterschenkels während der Schwungphase, in der der Unterschenkel pendelartig zurück- und vorschwingt, während das andere Bein den Körper unterstützt.

Eine Schwenkverbindung der eingangs beschriebenen Ausführungsform läßt sich der EP 0 482 809 A1 entnehmen. Offenbart ist hier eine fünfgliedrige Getriebekette bestehend aus einem Gelenkoberteil, einem Gelenkunterteil, einem streckseitig angeordneten Lenker sowie zwei beugeseitig angeordneten Lenkern. Diese Getriebekette weist zwei Freiheitsgrade auf, besitzt also keinen Zwangslauf. Diese Schwenkverbindung verfügt ferner über ein längenveränderliches Steuerelement, das mit seinem einen Ende am Gelenkoberteil und mit seinem anderen Ende an dem oberen der beiden beugeseitig angeordneten Lenkern angelenkt ist. Diese beiden Anlenkpunkte haben bezogen auf das zugeordnete Anlenk-Gelenkglied eine ortsfeste Position.

Der Erfindung liegt die Aufgabe zugrunde, ein Gelenk der eingangs beschriebenen Ausführungsform mit verbesserten Eigenschaften zur Verfügung zu stellen.

In Verbindung mit den eingangs beschriebenen Gelenkmerkmalen wird diese Aufgabe erfindungsgemäß dadurch gelöst, daß zumindest einer der genannten Anlenkpunkte seine Lage gegenüber seinem Anlenk-Gelenkglied durch getriebetechnische Zwangskopplung mit zumindest einem anderen Gelenkglied in Abhängigkeit von der Gelenkbewegung oder Verschwenkung selbsttätig verändert.

Dabei kann die Lageveränderung zumindest eines der genannten Anlenkpunkte auf einer Kreisbahn um eine an dem Anlenk-Gelenkglied vorgesehene Drehachse erfolgen; die Lageveränderung kann aber auch eine Gleitbewegung entlang einer vorgegebenen Gleitbahn sein.

Die mechanische Zwangskopplung kann ein Stirnradgetriebe sein, das ein- oder mehrstufig ausgeführt ist, wobei eine Stufe als Zahnradgetriebe oder als Reibradgetriebe ausgeführt sein kann. Die Räder einer Stufe des Getriebes können als kämmendes oder aufeinander abrollendes Kurvenscheibenpaar ausgebildet sein. Weitere Varianten sind Gegenstand der Unteransprüche und werden anhand weiterer Ausführungsbeispiele erläutert.

Die Lageveränderung des zumindest einen Anlenkpunktes gegenüber seinem Anlenk-Gelenkglied kann entlang einer Bahn erfolgen, die bei konsequenter Nutzung aller sinnvollen getriebetechnischen Varianten je nach angestrebter Wirkung weitgehend frei konzipiert werden kann. Dadurch wird dem Konstrukteur erstmalig die Möglichkeit geboten, mehrere Einzelfunktionen unabhängig voneinander zu optimieren.

Grundsätzlich lassen sich direkte und indirekte Auswirkungen der erfindungsgemäßen Lageveränderung auf einzelne funktionelle Merkmale der genannten Gelenke voneinander unterscheiden:

Direkter Einfluß läßt sich erfindungsgemäß nehmen z. B. auf Größe und Verlauf des wirksamen Gesamt-Hebelarmes der Anlenkung in bezug auf die Kraftwirkung des Steuerelementes auf den Drehpunkt eines monozentrischen oder das Momentanzentrum der Bewegung eines polyzentrischen Gelenkes. Unmittelbar beeinflussen lassen sich auch Größe und Verlauf des Hubes am Steuerelement sowie die Lage der Wirkungsumkehr.

Indirekt beeinflussen lassen sich Gelenkmomente aus der Wirkung eines bewegungshemmenden Steuerelementes, das auf dem Prinzip der mechanischen Reibung arbeitet. Das gleiche gilt für Gelenkmomente aus der Wirkung eines federelastisch puffernden Steuerelementes sowie aus der Wirkung eines bewegungshemmenden Steuerelementes, dessen Wirkung von der Geschwindigkeit seiner translatorischen Bewegung abhängt (hierzu zählen insbesondere Fluid-Dämpfer).

Der indirekte Einfluß der erfindungsgemäßen Lageveränderung der Anlenkung auf die letztgenannte Gattung Steuerelemente ergibt sich aus folgendem Zusammenhang:

Der Hub am Steuerelement als direkt beeinflußbare Größe steht in kinematischer Abhängigkeit zur Winkelbewegung des betroffenen Gelenkes. Für bestimmte Bewegungsmuster der menschlichen Extremitäten stehen die Winkelbewegungen in Abhängigkeit zur Zeit, d. h. sie weisen ein charakteristisches Winkelgeschwindigkeits-Profil auf. Steuerelemente in Gestalt von Fluid-Dämpfern, die in ein und demselben Typ eines Gelenkes eines orthopädischen Hilfsmittels, z. B. im Kniegelenk einer Beinprothese, eingesetzt werden, um die natürliche Winkelbewegung möglichst nachzubilden, können diese Aufgabe bei vorgegebenem Winkelgeschwindigkeits-Profil des Gelenkes erfahrungsgemäß nur dann erfüllen, wenn gleichzeitig auch das Niveau ihrer Kolbenstangen-Geschwindigkeit hoch genug liegt. Dazu bedarf es eines Mindesthubes, der bei den herkömmlichen Ausführungsformen kaum erreicht werden kann, wenn gleichzeitig weitere funktionelle Anforderungen gestellt werden. Durch gezielte Anwendung des erfindungsgemäßen Konzeptes der gelenkwinkelabhängigen Lageveränderung der Anlenkung gelingt es, durch Vergrößerung des Hubes und Anpassung des Hubverlaufes als direkte Einflußnahme gleichzeitig als indirekte Auswirkung auch die Hubgeschwindigkeit in gewünschter Weise zu erhöhen.

Ein monozentrisches Prothesen-Kniegelenk kann erfindungsgemäß dadurch gekennzeichnet sein, daß das Gelenkunterteil drehfest verbunden ist mit einem koaxial zur Gelenkachse angeordneten ersten Stirnrad, das mit einem zweiten Stirnrad im Eingriff steht, das mit seiner Drehachse achsparallel zur Gelenkachse vor (streckseitig) oder hinter (beugeseitig) dieser am Gelenkoberteil drehbar gelagert ist und einen Kurbelarm aufweist, an dem das Steuerelement über den zumindest einen Anlenkpunkt angelenkt ist.

Ein polyzentrisches Prothesen-Kniegelenk in Form einer viergliedrigen kinematischen Gelenkkette, bei der das Gelenkoberteil mit dem Gelenkunterteil über einen vorderen (streckseitigen) und einen hinteren (beugeseitigen) Lenker verbunden ist, kann erfindungsgemäß so gestaltet werden, daß der vordere Lenker koaxial zu seiner oberen, ihn mit dem Gelenkoberteil verbindenden Gelenkachse mit einem ersten Stirnrad drehfest verbunden ist, das mit einem zweiten Stirnrad im Eingriff steht, das drehbar gelagert ist auf der oberen, den hinteren Lenker mit dem Gelenkoberteil verbindenden Gelenkachse und einen Kurbelarm aufweist, an dem das Steuerelement über den zumindest einen Anlenkpunkt angelenkt ist.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung sowie Beispiele zum Stand der Technik dargestellt. Es zeigen:
- Figur 1 -: in schematischer Darstellung eine beuge- und streckseitige obere Anlenkung eines als Zylinder mit Kolbenstange ausgebildeten Steuerelementes in einem monozentrischen Gelenk mit Hilfe eines einstufigen Stirnradgetriebes;
- Figur 2 -: in Draufsicht eine beugeseitige obere Anlenkung eines nicht näher dargestellten Steuerelementes in einem monozentrischen Gelenk mit Hilfe eines Planetengetriebes;
- Figur 3 -: einen Querschnitt gemäß der Linie III-III in Figur 2;
- Figur 4 -: in schematischer Darstellung ein als viergliedrige kinematische Kette ausgebildetes polyzentrisches Prothesen-Kniegelenk mit einem diagonal über zwei Doppelkurbeltriebe angelenkten Steuerelement;
- Figur 5 -: für das Steuerelement in Figur 4 den Verlauf des Hubes, des wirksamen Hebelarmes und des Gelenkmomentes in Abhängigkeit vom Kniebeugewinkel;
- Figur 6 -: ein allgemeines Schema zur Anwendung des erfindungsgemäßen Konzeptes;
- Figur 7 -: ein Schema gemäß Figur 6 mit beispielhafter Angabe von Wirkungen bei beuge- und streckseitiger oberer Anlenkung eines Steuerelementes;
- Figur 8 -: eine Darstellung gemäß Figur 7 für eine beuge- und streckseitige untere Anlenkung eines Steuerelementes;
- Figur 9 -: in schematischer Darstellung eine beugeseitige obere Anlenkung einer Koppelstange eines als Doppel-Schubkolbentriebs ausgebildeten Fluid-Dämpfers in einem polyzentrischen Gelenk mit Hilfe eines Doppelkurbeltriebes;
- Figur 10 -: in einer Darstellung gemäß Figur 9 eine beugeseitige obere Anlenkung eines als Zylinder mit Kolbenstange ausgebildeten Steuerelementes in einem polyzentrischen Gelenk mit Hilfe eines Schubkurbeltriebes mit äußerem Schubglied;
- Figur 11 -: in einer Darstellung gemäß Figur 10 eine beugeseitige obere Anlenkung eines als Zylinder mit Kolbenstange ausgebildeten Steuerelementes in einem monozentrischen Gelenk mit Hilfe eines Schubkurbeltriebes mit innerem Schubglied;
- Figur 12 -: in einer Darstellung gemäß Figur 11 eine beidseitig gelenkwinkelabhängige lageveränderliche Anlenkung eines als Zylinder mit Kolbenstange ausgebildeten Steuerelementes für zwei monozentrische Gelenke mit Hilfe zweier einstufiger Stirnradgetriebe;
- Figur 13 -: in Draufsicht ein monozentrisches Prothesen-Kniegelenk mit gelenkwinkelabhängiger Lageveränderung der beugeseitigen oberen Anlenkung eines nicht näher dargestellten Steuerelementes mit Hilfe eines einstufigen Stirnradgetriebes;
- Figur 14 -: einen Schnitt gemäß der Linie XIV-XIV in Figur 13
- Figur 15 -: in Seitenansicht ein polyzentrisches Prothesen-Kniegelenk mit gelenkwinkelabhängiger Lageveränderung der beugeseitigen oberen Anlenkung einer Koppelstange eines nicht näher dargestellten Steuerelementes mit Hilfe eines einstufigen Stirnradgetriebes;
- Figur 16 -: das Kniegelenk gemäß Figur 15 in Draufsicht;
- Figur 17 -: in vergrößertem Maßstab das Kniegelenk gemäß Figur 16 zum Teil im Längsschnitt;
- Figur 18 -: als Stand der Technik eine schematisch dargestellte Anlenkung eines als Zylinder mit Kolbenstange ausgebildeten Steuerelementes in fester Anlenkposition bei einem monozentrischen Gelenk und
- Figur 19 -: zum Stand der Technik in einer Darstellung gemäß Figur 4 ein polyzentrisches Gelenk mit zwei fest angelenkten diagonalen Steuerelementen jeweils in Form eines Zylinders mit Kolbenstange.

Zum besseren Verständnis soll das der Erfindung zugrundeliegende Problem vorab unter Bezugnahme auf den Stand der Technik erläutert werden:

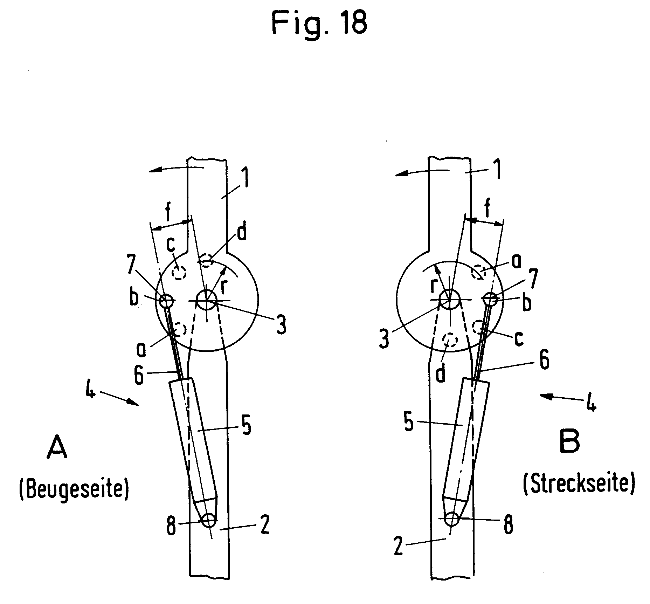

Figur 18 zeigt in der linken Darstellung A ebenso wie in der rechten Darstellung B jeweils ein Gelenk bzw. eine Schwenkverbindung bestehend aus einem Gelenkoberteil 1, einem Gelenkunterteil 2 und einer diese beiden Teile 1,2 verschwenkbar miteinander verbindenden Gelenkachse 3, die fest mit dem Gelenkunterteil 2 verbunden ist. Es handelt sich also um ein monozentrisches Gelenk bzw. eine monozentrische Schwenkverbindung, jeweils dargestellt in gestreckter Position. Vorgesehen ist ferner ein Steuerelement 4, das in den beiden Ausführungsbeispielen jeweils durch einen Zylinder 5 mit einer Kolbenstange 6 gebildet wird. Dieses längenveränderliche Steuerelement 4 ist an seinem oberen Ende am Gelenkoberteil 1 und mit seinem unteren Ende am Gelenkunterteil 2 angelenkt. Dabei ist der obere Anlenkpunkt mit 7 und der untere Anlenkpunkt mit 8 bezeichnet.

Bei der linken Darstellung A liegt der obere Anlenkpunkt 7 auf der Beugeseite des Gelenkes und somit hinter dessen Gelenkachse 3, bei der rechten Darstellung B hingegen auf der Streckseite des Gelenkes und somit vor der Gelenkachse 3. Das Steuerelement 4 liegt mit seiner Längsachse bei der linken Darstellung A somit insgesamt beugeseitig, bei der rechten Darstellung B hingegen streckseitig. Dadurch führt die Einleitung einer Beugung des Gelenkes z. B. durch Schwenkung des Gelenkoberteils 1 in Pfeilrichtung nach hinten bei der Ausführungsform gemäß A zu einer Verkleinerung des Abstandes der beiden Anlenkpunkte 7,8 voneinander, nämlich zu einer Einwärtsbewegung der Kolbenstange 6 in den Zylinder 5, während die gleiche Beugung bei der Ausführungsform B zu einer Vergrößerung des Abstandes der beiden Anlenkpunkte 7,8 voneinander führt, also zu einer Auswärtsbewegung der Kolbenstange 6 aus dem Zylinder 5.

Die Position des oberen Anlenkpunktes 7 am Gelenkoberteil 1 bezogen auf die Gelenkachse 3 ist maßgeblich dafür, ob die Anlenkseite des Steuerelementes 4 bei Fortsetzung der Beugebewegung des Gelenkes im konstruktiv möglichen Bereich wechselt, d. h. ob die Längsachse des Steuerelementes 4 bei der Ausführungsform gemäß A aus einer Position hinter der Gelenkachse 3 in eine Position vor dieser Gelenkachse 3 gerät bzw. ob die Längsachse des Steuerelementes 4 bei der Ausführungsform gemäß B aus einer Position vor der Gelenkachse 3 in eine Position hinter diese Gelenkachse 3 gerät. Tritt ein entsprechender Wechsel ein, kehrt sich zugleich auch die Richtung der translatorischen Bewegung der Kolbenstange 6 zum Zylinder 5 und damit auch die Richtung seiner in dieser Stellung vorhandenen Wirkung nach vorherigem Stillstand bzw. vorherigem Nulldurchgang um (nachfolgend als Wirkungsumkehr mit "WU" bezeichnet). Dies kann je nach der Funktion des Steuerelementes 4 zu unterschiedlichen Wirkungen auf das Gelenk führen: Ein federelastisch pufferndes System, das bis zum Erreichen der WU-Grenze der Gelenkbewegung entgegenwirkt, wird letztere nach Überschreiten der WU-Grenze unterstützen. Möglich ist naturgemäß auch die umgekehrte Reihenfolge der Wirkungen.

Ein bewegungshemmendes System kann nach Überschreiten der WU-Grenze ebenfalls unterschiedliche Wirkungen auf das Gelenk ausüben: Bei Funktion in beiden Bewegungsrichtungen wird dieses bewegungshemmende System (Steuerelement) der Gelenkbewegung auch weiterhin entgegenwirken ggf. mit unterschiedlicher Widerstandscharakteristik. Bei Funktion in nur einer Bewegungsrichtung wird das Steuerelement wirkungslos, wobei wie beim federelastisch puffernden System auch in diesem Fall die umgekehrte Reihenfolge der Wirkungen möglich ist.

Diese Wirkungsumkehr tritt je nach Position des oberen Anlenkpunktes 7 am Gelenkoberteil 1 (bezogen auf die Gelenkachse 3) bei unterschiedlichen Beugewinkeln ein, und zwar immer dann, wenn die Längsachse des Steuerelementes 4 die Gelenkachse 3 schneidet. Dies ereignet sich für die vier eingezeichneten oberen Anlenkpunkte a,b,c und d bei beiden Ausführungsformen A und B einheitlich annähernd bei folgenden Beugewinkeln: 45° für a, 90° für b, 135° für c und 180° für d. Für monozentrische Prothesen-Kniegelenke sind von diesen Anlenkmöglichkeiten für beuge- und streckseitig angelenkte Steuerelemente 4 praktisch nur die Anlenkvarianten b und c von Bedeutung, wobei die Variante b mit Wirkungsumkehr bei etwa 90° in der Regel bevorzugt wird. Ist nämlich in dem Zylinder 5 ein federelastisches Element angeordnet, das bei Einleitung einer Beugung des Gelenkes aus der Strecklage gespannt wird, so erleichtert seine Wirkungsumkehr bei einem Beugewinkel von etwa 90° in einem Prothesen-Kniegelenk das Sitzen des Amputierten, da die streckende Wirkung des gespannten federelastischen Elementes auf den Unterschenkel bei diesem Winkel gerade Null ist und sich beim Überschreiten des Winkels umkehrt, also die Beugestellung unterstützt.

Dennoch ist die Anlenkvariante b mit zum Teil gravierenden Nachteilen verbunden: Die Position des oberen Anlenkpunktes 7 bezogen auf die Gelenkachse 3 hat auch maßgeblichen Einfluß auf Ausmaß und Verlauf der translatorischen Bewegung der Kolbenstange 6 gegenüber dem Zylinder 5 in Abhängigkeit vom Beugewinkel des Gelenkes. Außerdem bestimmt die genannte Position den Verlauf des lotrechten Abstandes f der Längsachse des Steuerelementes 4 von der Gelenkachse 3 in Abhängigkeit vom Beugewinkel des Gelenkes. Dieser Abstand f ist das Maß für den wirksamen Hebelarm, an dem Kräfte zwischen Steuerelement 4 und Gelenk übertragen werden und z. B. als Kniemoment wirken.

Geht man gemäß den beiden Ausführungsbeispielen in Figur 18 davon aus, daß die vier als möglich angesehenen Anlenkpunkte a - d auf einem Kreisbogen mit dem Radius r um die Gelenkachse 3 angeordnet sind, dann ergibt sich aus Figur 18 unmittelbar, daß innerhalb eines Gelenkbeugebereiches von 0° bis 180° der Maximalwert der als Hub (H) bezeichneten translatorischen Bewegung der Kolbenstange 6 zum Zylinder 5 den doppelten Wert des Anlenkradius r und der Maximalwert des wirksamen Hebelarmes f den einfachen Wert des Anlenkradius r nicht überschreiten können. Maßgebend für die Wirkung der Steuerelemente bzw. Steuersysteme sind somit Hub H und wirksamer Hebelarm f, die bei endoskelettalen Prothesenkomponenten dem Konstrukteur bisher relativ enge Bewegungsgrenzen gesetzt haben.

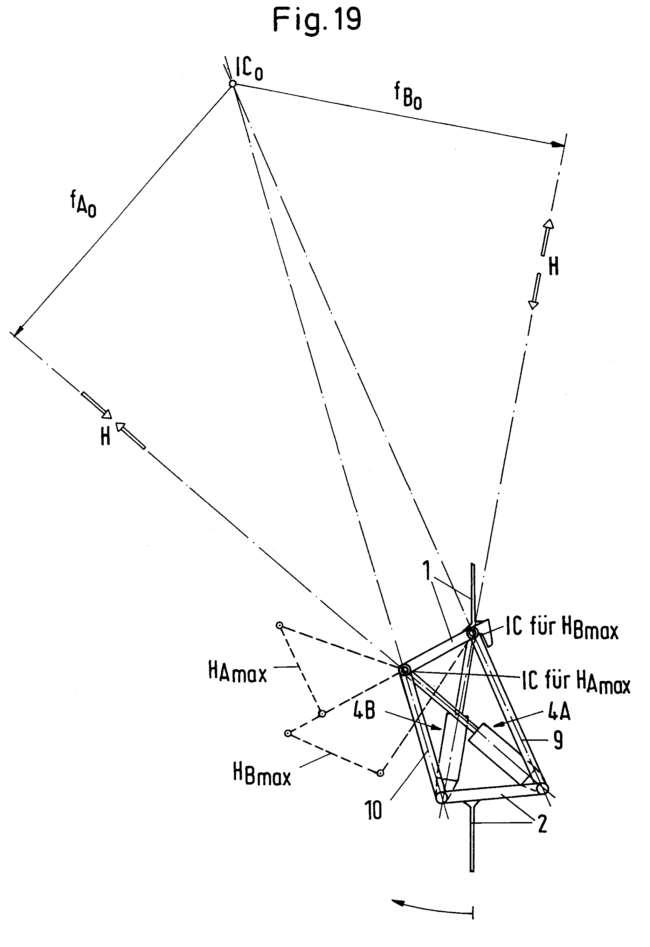

Zum Stand der Technik zeigt Figur 19 ein polyzentrisches Gelenk bzw. eine polyzentrische Schwenkverbindung in Form einer viergliedrigen kinematischen Gelenkkette, bei der das Gelenkoberteil 1 mit dem Gelenkunterteil 2 über einen vorderen (streckseitigen) Lenker 9 und einen hinteren (beugeseitigen) Lenker (10) verbunden ist. Eingezeichnet sind zwei unterschiedliche, jeweils diagonal angelenkte Steuerelemente 4A und 4B, wobei es sich jeweils um eine direkte bzw. unmittelbare Anlenkung zwischen zwei benachbarten Gliedern eines Gelenkes handelt. Dabei entspricht die Anordnung des Steuerelementes 4A angenähert einem Knie-Exartikulationsgelenk gemäß DE 28 41 999 C2. Bezüglich der Relativbewegungen zwischen Zylinder und Kolbenstange bei Einleitung einer Beugebewegung des Gelenkes aus der Strecklage gelten die entsprechenden vorstehenden Erläuterungen zur Figur 18 mit dem Unterschied, daß für die Richtung dieser Relativbewegungen nicht die Lage der die Längsachse der Steuerelemente 4 darstellenden Verbindungsgeraden durch ihre Anlenkpunkte zur Gelenkachse, sondern zum Momentanzentrum IC (IC = Instantaneous Centre) der Bewegung des Gelenkes maßgebend ist.

Sowohl die Konfiguration gemäß Steuerelement 4A als auch die gemäß Steuerelement 4B weisen in mehrfacher Hinsicht Mängel auf: Der Maximalwert des wirksamen Hebelarmes f tritt bereits in der Strecklage auf. Der Maximalhub Hₘₐₓ ist nicht sehr groß. Die Wirkungsumkehr tritt für die Verwendung als Prothesen-Kniegelenk oder Prothesen-Hüftgelenk bei der Konfiguration B bei etwa 30° und bei der Konfiguration A bei etwa 70° und damit zu früh, d. h. bei zu geringen Beugewinkeln ein.

Zur Lösung dieser vorstehend anhand des Standes der Technik erläuterten Probleme dienen die nachfolgend beispielsweise beschriebenen Ausführungsformen der Erfindung:

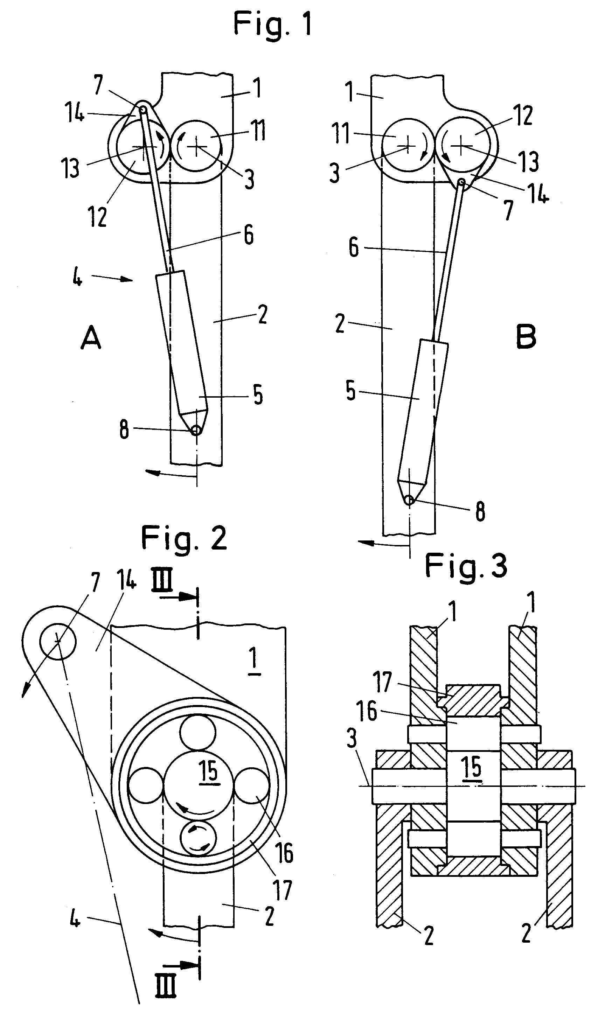

Figur 1 zeigt der Figur 18 analoge Darstellungen. Dargestellt sind wieder zwei monozentrische Gelenke mit jeweils einem Gelenkoberteil 1, einem Gelenkunterteil 2, einer Gelenkachse 3 und einem Steuerelement 4, das wiederum durch einen Zylinder 5 mit Kolbenstange 6 gebildet wird. Bei der linken Darstellung A ist das Steuerelement in Übereinstimmung mit Figur 18A beugeseitig und bei der rechten Darstellung B gemäß Figur 18B streckseitig angeordnet. Der obere Anlenkpunkt des Steuerelementes 4 ist wiederum mit dem Bezugszeichen 7 und der untere Anlenkpunkt mit dem Bezugszeichen 8 gekennzeichnet.

Erfindungsgemäß ist jedoch der obere Anlenkpunkt 7 gegenüber dem Gelenkoberteil 1 (Anlenk-Gelenkglied) lageveränderlich angeordnet und über eine mechanische Zwangskopplung mit dem Gelenkunterteil 2 kinematisch verbunden. Dabei ist die mechanische Zwangskopplung gemäß Figur 1 als einstufiges Stirnradgetriebe ausgebildet, bei dem das erste Stirnrad 11 drehfest auf der mit dem Gelenkunterteil 2 fest verbundenen Gelenkachse 3 sitzt und in ein zweites Stirnrad 12 eingreift, das auf einer zusätzlichen Drehachse 13 frei drehbar am Gelenkoberteil 1 gelagert ist und einen Kurbelarm 14 aufweist, an dem das Steuerelement 4 über seinen oberen Anlenkpunkt 7 angelenkt ist. Bei der linken Darstellung A liegt die Drehachse 13 für das zweite Stirnrad 12 hinter der Gelenkachse 3 und bei der Ausführungsform B vor der Gelenkachse 3.

Bei einer Beugebewegung des Gelenkunterteils 2 in Pfeilrichtung führt das zweite Stirnrad 12 eine dieser Beugebewegung immer entgegengerichtete Drehbewegung aus. Der obere Anlenkpunkt 7 des Steuerelementes 4 beschreibt dabei eine Schwenkung entlang einer mit annähernd gleichen Anteilen nach hinten und unten gerichteten und um die Gelenkachse 3 verlaufenden Kreisbahn, die zur Verkürzung des Abstandes zwischen den beiden Anlenkpunkten 7,8 des Steuerelementes 4 bei Einleitung einer Kniebeugung aus der Strecklage und damit zur Vergrößerung des Hubes am Steuerelement 4 beiträgt. Der dabei erreichte Schwenkwinkel hängt allein vom Übersetzungsverhältnis des Stirnradpaares 11,12 ab und ist daher wie der Anlenkradius des Kurbelarmes 4 frei wählbar.

Bei dem Ausführungsbeispiel gemäß Figur 2 wird die erfindungsgemäße mechanische Zwangskopplung zwischen dem Anlenkpunkt 7 und dem Gelenkunterteil 2 durch ein koaxial zur Gelenkachse 3 angeordnetes Planetengetriebe erreicht, bei dem das zentrale Sonnenrad 15 mit dem Gelenkunterteil 2 und der Träger der Planetenräder 16 mit dem Gelenkoberteil 1 gekoppelt sind. Bei dieser Anordnung führt das äußere Hohlrad 17 eine der Beugebewegung des Gelenkunterteils 2 immer entgegengerichtete Drehbewegung aus. Der mit dem äußeren Hohlrad 17 verbundene Kurbelarm 14 trägt an seinem Ende den oberen Anlenkpunkt 7 für das Steuerelement 4. Dieser obere Anlenkpunkt 7 beschreibt bei der genannten Beugebewegung eine Schwenkung entlang einer geringfügig nach hinten und überwiegend nach unten gerichteten sowie konzentrisch zur Gelenkachse 3 verlaufenden Kreisbahn, die zur Verkürzung des Abstandes zwischen den beiden Anlenkpunkten des Steuerelementes 4 bei Einleitung einer Kniebeugung aus der Strecklage und damit zur Vergrößerung des Hubes am Steuerelement beiträgt. Der dabei erreichte Schwenkwinkel hängt allein vom Übersetzungsverhältnis zwischen Hohlrad 17 und Sonnenrad 15 ab und ist daher ebenso frei wählbar wie der Anlenkradius des Kurbelarmes 14.

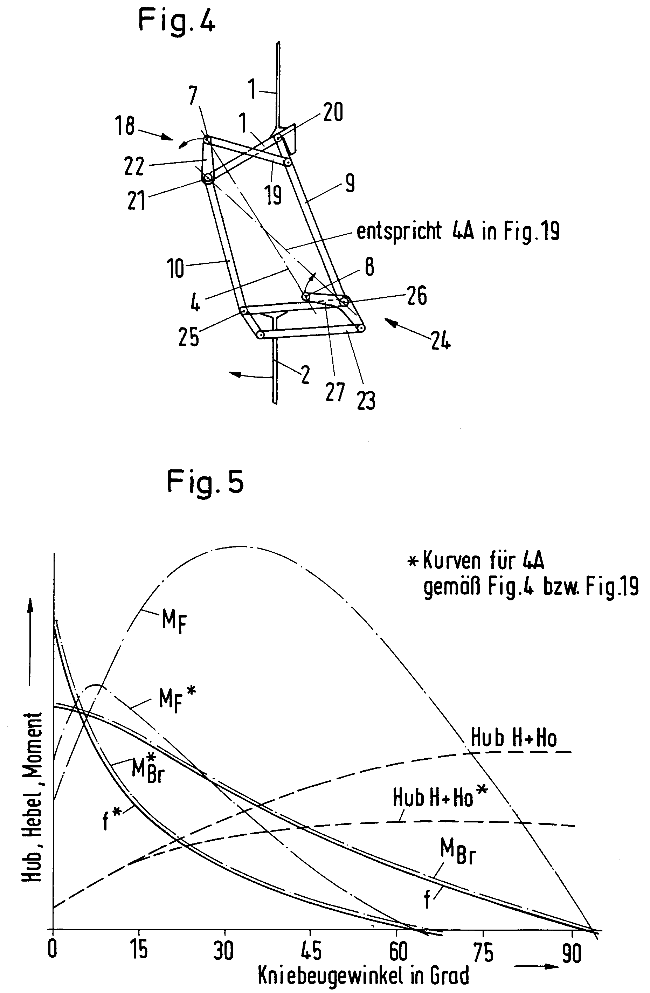

Figur 4 zeigt die erfindungsgemäße Abwandlung des in Figur 19 zum Stand der Technik dargestellten polyzentrischen Gelenkes bestehend aus einer viergliedrigen kinematischen Gelenkkette, die sich zusammensetzt aus dem Gelenkoberteil 1, dem Gelenkunterteil 2, dem vorderen Lenker 9 und dem hinteren Lenker 10. Das diagonal zwischen dem oberen Anlenkpunkt 7 und dem unteren Anlenkpunkt 8 eingehängte Steuerelement 4 ist lediglich schematisch durch eine strichpunktierte Linie angedeutet. Die Längsachse des Steuerelementes 4 verläuft somit angenähert diagonal von hinten oben nach vorn unten. Die Anlenkung erfolgt jeweils über einen Doppelkurbeltrieb. Der obere Doppelkurbeltrieb 18 ist über seine Koppelstange 19 unterhalb des vorderen oberen Gelenkes 20 der Grundmechanik am vorderen Lenker 9 angelenkt und treibt einen koaxial zum hinteren oberen Gelenk 21 der Grundmechanik gelagerten, nach oben weisenden Hebel 22 über ein Gelenk, zu dem der obere Anlenkpunkt 7 des Steuerelementes 4 koaxial angeordnet ist.

Die Koppelstange 23 des unteren Doppelkurbeltriebes 24 ist unterhalb des hinteren unteren Gelenkes 25 der Grundmechanik an dem nach unten verlängerten hinteren Lenker 10 angelenkt und treibt einen koaxial zum vorderen unteren Gelenk 26 der Grundmechanik gelagerten Winkelhebel 27, an dessen gegenüberliegendem Ende sich der untere Anlenkpunkt 8 des Steuerelementes 4 befindet.

Zum Vergleich ist in Figur 4 das in Figur 19 zum Stand der Technik eingezeichnete Steuerelement 4A ebenfalls in einer strichpunktierten Linie dargestellt.

Hub-, Hebel- und Moment-Verlauf sind in Figur 5 über den Kniebeugewinkel eines Kniegelenkes gemäß Figur 4 aufgetragen. Figur 5 läßt deutlich erkennen, daß alle dargestellten Größen gegenüber den zum Vergleich eingezeichneten Kurven für ein bekanntes Gelenk gemäß Figur 19 deutlich besser ausfallen. Die Wirkungsumkehr ist von der für ein Prothesen-Kniegelenk viel zu geringen Beugestellung von etwa 60° zur zweckmäßigen Beugestellung von etwa 90° verlagert. Der rechnerisch wirksame Gesamt-Hebelarm hat einen wesentlich günstigeren Verlauf. Der Hub H ist gegenüber der Vergleichsgröße erheblich verstärkt und zeigt mit dem Maximalwert bei etwa 90° Gelenkbeugung einen zweckmäßigen Verlauf. Das Moment M_{F} aus der Wirkung eines federelastischen Elementes 4 ist aufgrund der Steigerung des Hubes bei größeren Beugewinkeln sowie des günstigeren Verlaufes des rechnerisch wirksamen Gesamt-Hebelarmes über den gesamten Beugebereich erheblich verstärkt und erreicht sein Maximum deutlich später als die denkbar ungünstig verlaufende Vergleichsgröße. Das Moment M_{Br} aus der Wirkung eines bewegungshemmenden Elementes folgt - da proportional abhängig - dem Verlauf des rechnerisch wirksamen Gesamt-Hebelarmes. Die entsprechenden Ausführungen gelten daher sinngemäß.

Zur besseren Übersicht der vielfältigen Möglichkeiten hinsichtlich der Anlenkung des Steuerelementes ist es zweckmäßig, für den Bahnverlauf der erfindungsgemäßen gelenkwinkelabhängigen Lageveränderung des oberen Anlenkpunktes eines Steuerelementes gegenüber dem Anlenk-Gelenkglied eine Differenzierung in einzelne charakteristische Bahnkomponenten vorzunehmen. Hierzu bietet sich die Orientierung an der Längsachse des orthopädie-technischen Hilfsmittels an, zumal diejenigen orthopädischen Prothesen und Orthesen, in denen das erfindungsgemäße Konzept der lageveränderlichen Anlenkung von Steuerelementen Anwendung finden kann, in der Regel eine auf ihre Grund- oder Ausgangsstellung innerhalb des menschlichen Stütz- und Bewegungsapparates bezogene Längsachse aufweisen. Folgt man dieser Orientierung, bietet sich ein kartesisches Koordinatensystem an, dessen Ordinate mit L (Längsachse) und dessen Abszisse mit Q_{BE} (Querachse in der Bewegungsebene) bezeichnet werden. Die positive L-Achse zeigt nach oben, die positive Q_{BE}-Achse zur Streckseite des Gelenkes. Der Koordinatenursprung wird bei monozentrischen Gelenktypen auf den Gelenkmittelpunkt und bei polyzentrischen Gelenken auf einen zweckmäßigen Referenzpunkt gelegt (z. B. die Mitte einer von mehreren prominenten Gelenkachsen).

Bei Einordnung des jeweils zu konstruierenden oder zu bewertenden Bahnverlaufes der erfindungsgemäßen gelenkwinkelabhängigen Lageveränderung der Anlenkung von Steuerelementen in Gelenken orthopädischer Prothesen und Orthesen in dieses Bezugssystem ist es relativ einfach, entweder mit Hilfe in L- und Q_{BE}-Richtung angegebener Wirkungen den für vorgegebene funktionelle Anforderungen zweckmäßigsten Bahnverlauf der Anlenkung in Abhängigkeit vom Gelenkwinkel zu ermitteln oder die parallel und in der Bewegungsebene quer zu ihrer Längsachse verlaufenden L- und Q_{BE}-Komponenten bekannter Bahnverläufe einzelnen Wirkungen zuzuordnen bzw. diese auf bestimmte Bahnkomponenten zurückzuführen.

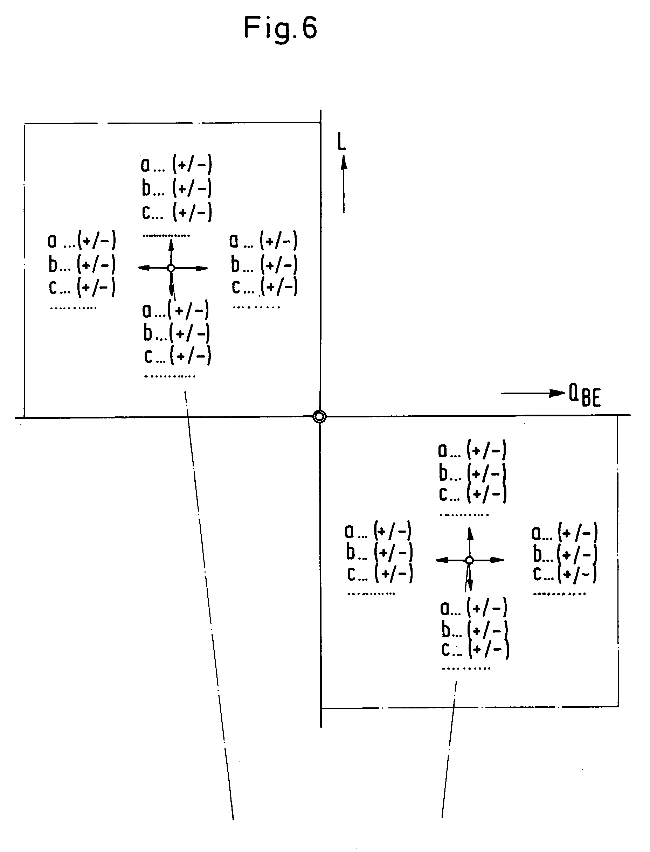

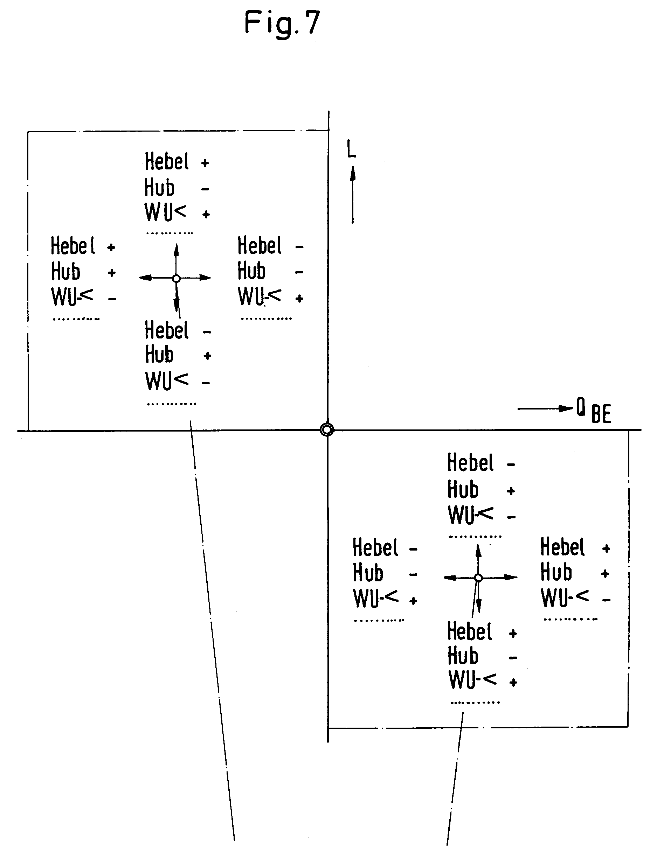

Dies ist in Figur 6 allgemein und Figur 7 speziell beispielhaft für die beuge- und streckseitige obere Anlenkung eines nicht näher dargestellten Steuerelementes in einem monozentrischen Prothesen-Kniegelenk dargestellt. In beiden Fällen sind die Anlenkpunkte im jeweiligen typischen Quadranten angeordnet. Für ein Prothesen-Hüftgelenk gelten dieselben Zusammenhänge. Die Darstellungen zeigen, welche Wirkungen einzeln oder in Kombination erzielbar sind, wenn sich der jeweils betrachtete Anlenkpunkt in Abhängigkeit von der Gelenkbewegung entlang der angestrebten oder vorgegebenen Bahn der Lageveränderung bei Einleitung einer Kniebeugung aus der Strecklage bewegt.

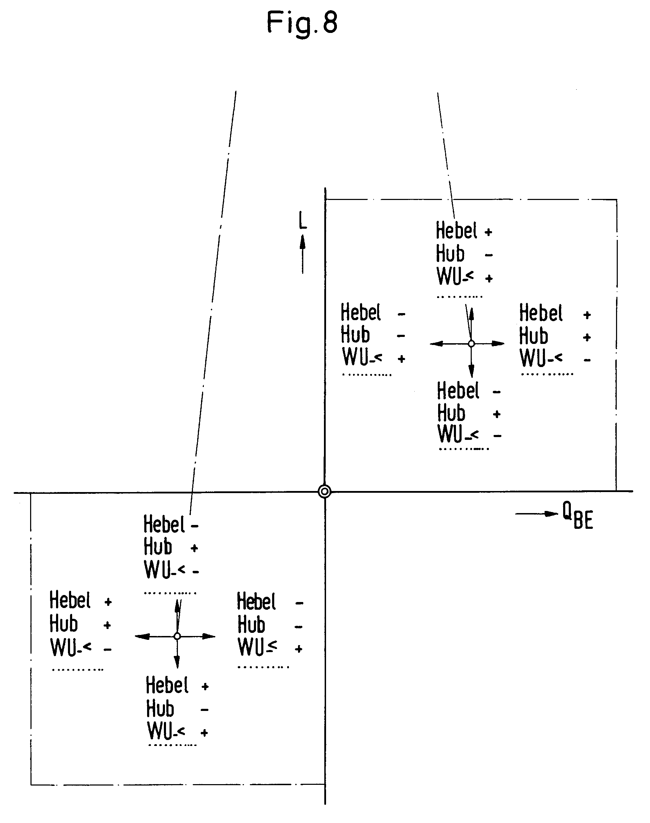

Der Vollständigkeit halber sind die in Figur 7 für die beuge- und streckseitige obere Anlenkung eines nicht näher dargestellten Steuergelenkes in einem monozentrischen Prothesen-Kniegelenk dargestellten Zusammenhänge in Figur 8 für die beuge- und streckseitige untere Anlenkung wiederholt.

Die nachfolgenden Figuren zeigen weitere beispielhafte Ausführungsformen der Erfindung:

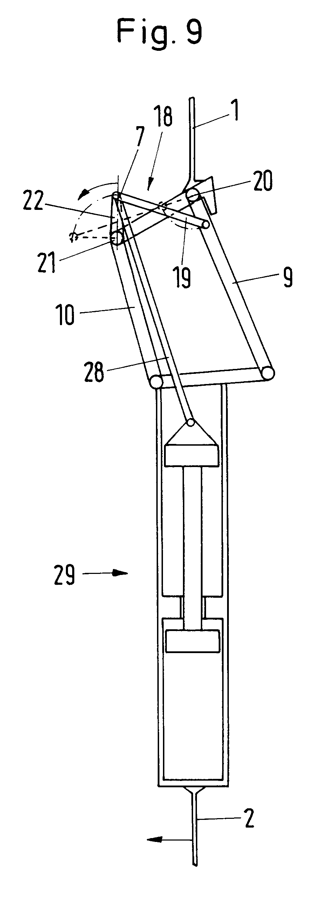

Figur 9 zeigt ein polyzentrisches Gelenk mit einem oberen Doppelkurbeltrieb 18 gemäß Figur 4. Am oberen Anlenkpunkt 7 ist beugeseitig die Koppelstange 28 eines als Doppel-Schubkolbentriebs ausgebildeten Fluid-Dämpfers 29 angelenkt.

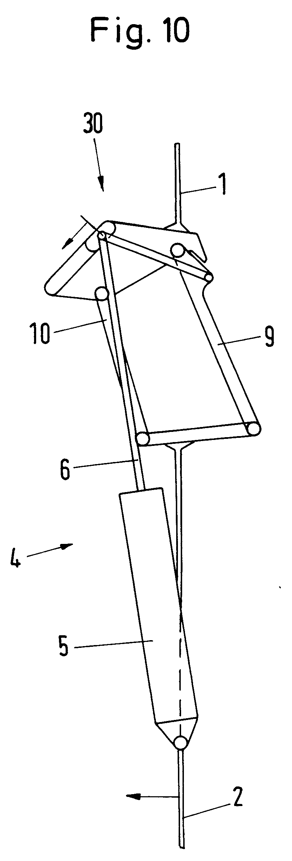

Figur 10 zeigt die gelenkwinkelabhängige Lageveränderung der beugeseitigen oberen Anlenkung eines als Zylinder 5 mit Kolbenstange 6 ausgebildeten Steuerelementes 4 in einem polyzentrischen Gelenk mit Hilfe eines Schubkurbeltriebes 30 mit äußerem Schubglied.

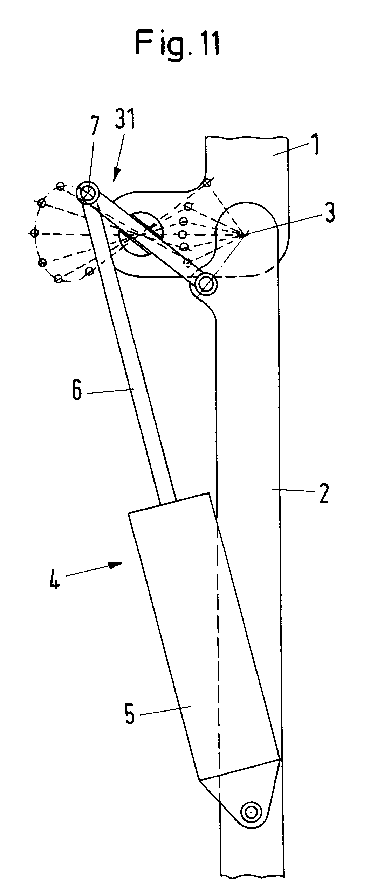

Figur 11 zeigt die gelenkwinkelabhängige Lageveränderung der beugeseitigen oberen Anlenkung eines als Zylinder 5 mit Kolbenstange 6 ausgebildeten Steuerelementes 4 in einem monozentrischen Gelenk mit Hilfe eines Schubkurbeltriebes 31 mit innerem Schubglied.

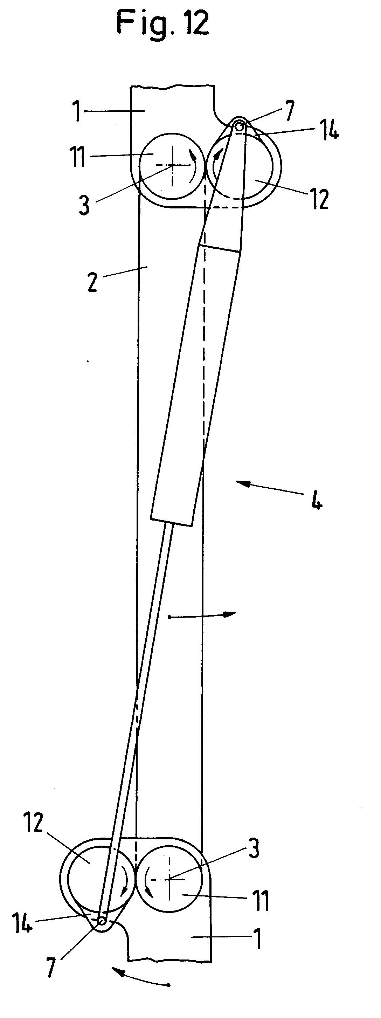

Figur 12 zeigt eine beidseitige gelenkwinkelabhängig lageveränderliche Anlenkung eines als Zylinder 5 mit Kolbenstange 6 ausgebildeten Steuerelementes 4 für zwei monozentrische Gelenke mit Hilfe zweier einstufiger Stirnradgetriebe.

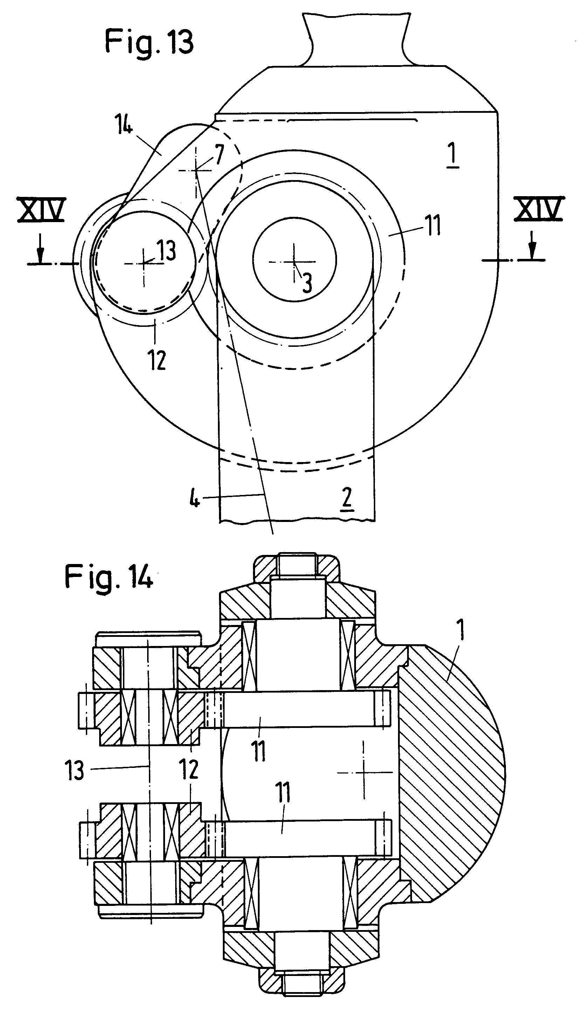

Die Figuren 13 und 14 zeigen ein monozentrisches Prothesen-Kniegelenk mit gelenkwinkelabhängiger Lageveränderung der beugeseitigen oberen Anlenkung 7 eines nicht näher ausgeführten Steuerelementes 4 mit Hilfe eines einstufigen Stirnradgetriebes. Hier ist das Gelenkunterteil 1 mit dem auf der Gelenkachse 3 gelagerten ersten Stirnrad 11 verbunden, so daß letzteres in der dargestellten Lage bei Einleitung einer Beugebewegung des Gelenkes aus der Strecklage durch Verschwenkung des Gelenkunterteils 2 im Uhrzeigersinn verdreht wird und dabei das mit ihm im Eingriff stehende zweite Stirnrad 12 im Gegenuhrzeigersinn um seine auf der Beugeseite des Gelenkes hinter dessen Gelenkachse 3 angeordnete Drehachse 13 treibt. Gleichsinnig mit dem zweiten Stirnrad 12 wird dessen Kurbelarm 14 und der an ihm befindliche obere Anlenkpunkt 7 verschwenkt. Dabei ist das Verhältnis der Teilkreisdurchmesser des aus den beiden Stirnrädern 11,12 gebildeten Zahnradpaares so gewählt, daß bei einem Gelenkbeugewinkel von 90° im Steuerelement 4 Wirkungsumkehr eintritt.

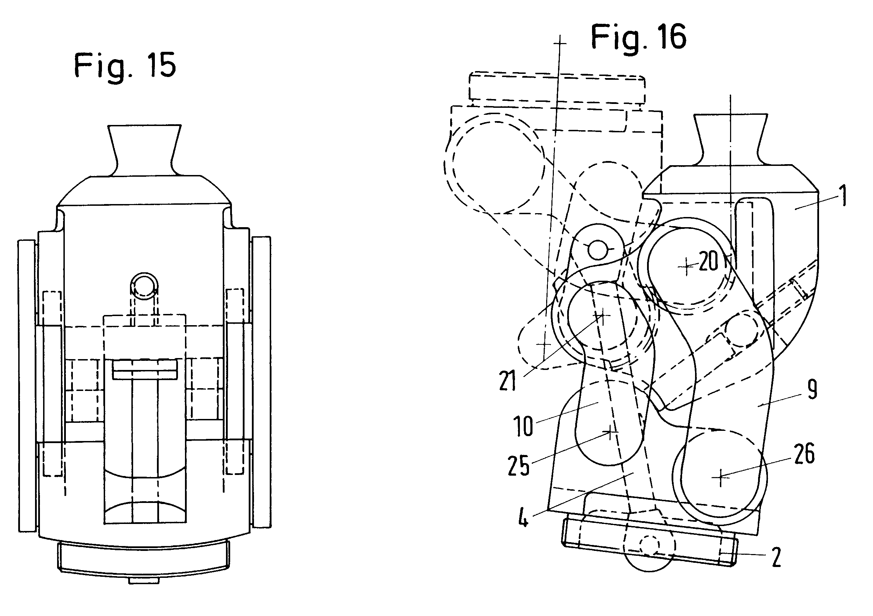

Bei dem in den Figuren 15, 16 und 17 schematisch dargestellten polyzentrischen Prothesen-Kniegelenk dient ebenfalls ein einstufiges Stirnradgetriebe zur mechanischen Zwangskopplung der Lageveränderung des oberen Anlenkpunktes 7 des Steuerelementes 4 im Gelenkoberteil 1 mit der Bewegung des als viergliedrige kinematische Kette gestalteten Gelenkes. Hierbei ist der vordere Lenker 9 in seinem vorderen oberen Gelenk 20 mit dem auf dessen Achse angeordneten ersten Stirnrad 11 verbunden, das bei Einleitung einer Beugebewegung des Gelenkes durch die damit verbundene Verschwenkung des vorderen Lenkers 9 um sein vorderes oberes Gelenk 20 im Uhrzeigersinn verdreht wird und das mit ihm im Eingriff stehende zweite Stirnrad 12 im Gegenuhrzeigersinn um seine koaxial zum hinteren oberen Gelenk 21 zwischen Gelenkoberteil 1 und dem hinteren Lenker 10 angeordnete Achse treibt, wobei der Kurbelarm 14 mit dem oberen Anlenkpunkt 7 gleichsinnig bewegt wird.

Das Verhältnis der Teilkreisdurchmesser des aus den Stirnrädern 11,12 gebildeten Zahnradpaares ist so gewählt, daß bei einem Gelenkbeugewinkel von 90° im Steuerelement 4 Wirkungsumkehr eintritt.

Besonders vorteilhaft ist bei dieser Ausführungsform eines polyzentrischen Gelenkes der mit annähernd 180° überdurchschnittlich große Kniebeugewinkel. Dieser wird dadurch ermöglicht, daß die obere Anlenkung 7 aus ihrer diesbezüglich hinderlichen Strecklagen-Position mit zunehmender Kniebeugung entlang einer Kreisbahn um ihre Schwenkachse nach hinten unten wegschwenkt und somit nicht hinderlich im Wege steht.

Konstruktive und funktionale Vorteile ergeben sich bei dieser Ausführungsform dann, wenn in Streckstellung die obere Gelenkachse 20 des vorderen Lenkers 9 oberhalb der oberen Gelenkachse 21 des hinteren Lenkers 10 und die untere Gelenkachse 26 des vorderen Lenkers 9 unterhalb der unteren Gelenkachse 25 des hinteren Lenkers 10 liegen, und wenn der Abstand l zwischen den beiden Gelenkachsen 21,25 des hinteren Lenkers 10 dem Abstand l zwischen den beiden Gelenkachsen 25,26 des Gelenkunterteils 2 entspricht.

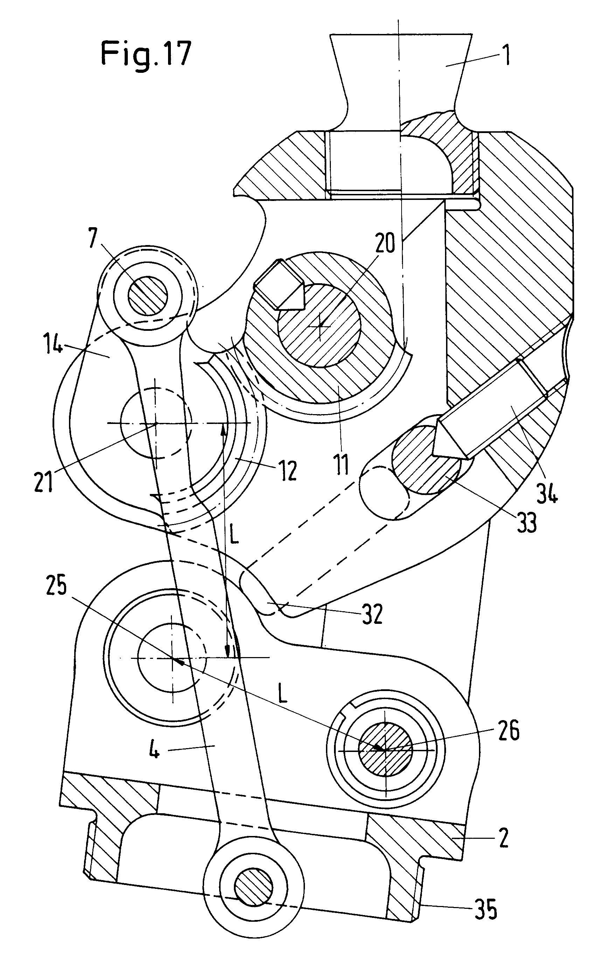

Vorteilhaft ist ferner, wenn die Streckstellung durch einen unmittelbar zwischen Gelenkoberteil 1 und Gelenkunterteil 2 wirkenden Streckanschlag definiert ist. Dabei besteht der Anschlag gemäß Figur 17 aus zwei parallel zueinander angeordneten, in den beiden Wangen des Gelenkoberteils geführten Anschlagpuffern 32, die über eine gemeinsame Traverse 33 von einer Einstellschraube 34 beaufschlagt werden. Der besondere Vorteil dieser konstruktiven Lösung ist darin zu sehen, daß beim Anschlag des Gelenkunterteils 2 gegen den bzw. die Anschlagpuffer 32 keine Biegemomente auf insbesondere den vorderen Lenker 9 ausgeübt werden. Außerdem können die Anschlagpuffer in dem als massives Bauteil ausgebildeten Gelenkoberteil 1 angeordnet werden.

Zum distalen Anschluß von Prothesenteilen mittels einer lösbaren Verbindung ist es vorteilhaft, wenn das Gelenkunterteil 2 zum Anschluß von Prothesenteilen eine lösbare Verbindung in Form eines Feingewindes 35 aufweist. Hierdurch kann das Gelenk werkseitig mit beliebigen Einheiten aus tragender Unterschenkelstruktur mit integriertem Steuerelement kombiniert werden.

## Patentansprüche

1. Gelenk oder Schwenkverbindungen in orthopädischen Prothesen und Orthesen, mit einem Gelenkoberteil (1), einem Gelenkunterteil (2) und in polyzentrischer Ausführung mit zusätzlich zwischengeschalteten Lenkern (9,10) (Gelenkober- und - unterteil sowie Lenker, nachfolgend zusammengefaßt als "Gelenkglieder" bezeichnet) und mit einem Steuerelement (4), das beidseitig über jeweils einen von einer Gelenkachse (3; 20, 21; 25, 26) beabstandeten Anlenkpunkt (7,8) wirkungsmäßig an jeweils einem Gelenkglied (11; 1,2) (nachfolgend "Anlenk-Gelenkglied") derart angreift, daß sich der Abstand zwischen seinen Anlenkpunkten (7,8) in Abhängigkeit von der Gelenkbewegung oder Verschwenkung verändert, wobei zumindest einer der genannten Anlenkpunkte (7,8) seine Lage gegenüber seinem Anlenk-Gelenkglied (1; 1,2) durch getriebetechnische Zwangskopplung (11,12,13,14; 15,16,17; 18,24; 30; 31) mit zumindest einem anderen Gelenkglied (2;9,10;9) in Abhängigkeit von der Gelenkbewegung oder Verschwenkung selbsttätig verändert.

2. Gelenk nach Anspruch 1, dadurch gekennzeichnet, daß die Lageveränderung zumindest eines der genannten Anlenkpunkte (7,8) auf einer Kreisbahn um eine an dem Anlenk-Gelenkglied (1; 9) vorgesehene Drehachse (13) erfolgt.

3. Gelenk nach Anspruch 2, dadurch gekennzeichnet, daß die Drehachse (13) koaxial zu einer prominenten Achse (3; 21) der Grundmechanik des Gelenkes verläuft.

4. Gelenk nach Anspruch 1, dadurch gekennzeichnet, daß die Lageveränderung des zumindest einen Anlenkpunktes (7,8) eine Gleitbewegung entlang einer vorgegebenen Gleitbahn ist.

5. Gelenk nach Anspruch 2 oder 3, dadurch gekennzeichnet, daß die mechanische Zwangskopplung ein einstufiges Stirnradgetriebe (11,12,13; 11,12,21) ist.

6. Gelenk nach Anspruch 2 oder 3, dadurch gekennzeichnet, daß die mechanische Zwangskopplung ein Planetengetriebe (15,16,17) ist.

7. Gelenk nach Anspruch 2 oder 3, dadurch gekennzeichnet, daß die mechanische Zwangskopplung ein Differentialgetriebe ist.

8. Gelenk nach einem der Ansprüche 1 bis 4, dadurch gekennzeichnet, daß die mechanische Zwangskopplung ein Stangentrieb ist.

9. Gelenk nach Anspruch 8, dadurch gekennzeichnet, daß der zumindest eine Anlenkpunkt (7) das Endglied eines Schubkurbeltriebes (30; 31) mit innerem oder äußerem Schubglied ist.

10. Gelenk nach Anspruch 8, dadurch gekennzeichnet, daß der Stangentrieb ein Doppelkurbeltrieb (18,24) mit Koppelstange (19,23) ist.

11. Gelenk nach Anspruch 1, 2 und 5 als monozentrisches Prothesen-Kniegelenk, dadurch gekennzeichnet, daß das Gelenkunterteil (2) drehfest verbunden ist mit einem koaxial zur Gelenkachse (3) angeordneten ersten Stirnrad (11), das mit einem zweiten Stirnrad (12) im Eingriff steht, das mit seiner Drehachse (13) achsparallel zur Gelenkachse (3) vor (streckseitig) oder hinter (beugeseitig) dieser am Gelenkoberteil (1) drehbar gelagert ist und einen Kurbelarm (14) aufweist, an dem das Steuerelement (4) über den zumindest einen Anlenkpunkt (7) angelenkt ist.

12. Gelenk nach Anspruch 11, dadurch gekennzeichnet, daß in der Streckstellung Gelenkachse (3) und Drehachse (13) in einer Ebene senkrecht zur Längsachse des orthopädie-technischen Hilfsmittels liegen.

13. Gelenk nach Anspruch 1, 2 und 5 als polyzentrisches Prothesen-Kniegelenk in Form einer viergliedrigen kinematischen Gelenkkette, wobei das Gelenkoberteil (1) mit dem Gelenkunterteil (2) über einen vorderen (streckseitigen) und einen hinteren (beugeseitigen) Lenker (9,10) verbunden ist, dadurch gekennzeichnet, daß der vordere Lenker (9) koaxial zu seiner oberen, ihn mit dem Gelenkoberteil (1) verbindenden Gelenkachse (20) mit einem ersten Stirnrad (11) drehfest verbunden ist, das mit einem zweiten Stirnrad (12) im Eingriff steht, das drehbar gelagert ist auf der oberen, den hinteren Lenker (10) mit dem Gelenkoberteil (1) verbindenden Gelenkachse (21) und einen Kurbelarm (14) aufweist, an dem das Steuerelement (4) über den zumindest einen Anlenkpunkt (7) angelenkt ist.

14. Gelenk nach Anspruch 11, 12 oder 13, dadurch gekennzeichnet, daß das Verhältnis der Wälz- oder Teilkreisdurchmesser der beiden Stirnräder (11,12) so gewählt ist, daß bei einem Gelenkbeugungswinkel von 90° im Steuerelement (4) Wirkungsumkehr eintritt.

15. Gelenk nach Anspruch 13 oder 14, dadurch gekennzeichnet, daß in Streckstellung die obere Gelenkachse (20) des vorderen Lenkers (9) oberhalb der oberen Gelenkachse (21) des hinteren Lenkers (10) und die untere Gelenkachse (26) des vorderen Lenkers (9) unterhalb der unteren Gelenkachse (25) des hinteren Lenkers (10) liegen.

16. Gelenk nach Anspruch 13, 14 oder 15, dadurch gekennzeichnet, daß der Abstand (l) zwischen den beiden Gelenkachsen (21,25) des hinteren Lenkers (10) dem Abstand (l) zwischen den beiden Gelenkachsen (25,26) des Gelenkunterteils (2) entspricht.

17. Gelenk nach einem der Ansprüche 13 bis 16, dadurch gekennzeichnet, daß die Streckstellung durch einen unmittelbar zwischen Gelenkoberteil (1) und Gelenkunterteil (2) wirkenden Streckanschlag (32,33,34) definiert ist.

18. Gelenk nach Anspruch 17, dadurch gekennzeichnet, daß der Streckanschlag (32,33,34) zwei parallel angeordnete, längsverschiebbare Anschlagpuffer (32) aufweist, die über eine gemeinsame Traverse (33) von einer Einstellschraube (34) beaufschlagt sind.

19. Gelenk nach einem der Ansprüche 13 bis 18, dadurch gekennzeichnet, daß das Gelenkunterteil (2) zum Anschluß von Prothesenteilen eine lösbare Verbindung in Form eines Feingewindes (35) aufweist.

## Claims

1. Joint or swivel connections in orthopaedic prostheses and orthotic devices, with a joint upper part (1), a joint lower part (2) and in polycentric design with additional intermediate guide rods (9, 10) (joint upper and lower parts and guide rods referred to below together as "joint members") and with a control element (4) which engages on both sides via a hinge point (7, 8) at a distance from a joint axis (3; 20, 21; 25, 26) to act on a joint member (11; 1, 2) (referred to below as a "hinge joint member"), such that the distance between its hinge points (7, 8) changes as a function of the joint movement or swivel, where at least one of the said hinge points (7, 8) automatically varies its position in relation to its hinge joint member (1; 1, 2) by a positive geared coupling (11, 12, 13, 14; 15, 16, 17; 18, 24; 30; 31) with at least one other joint member (2; 9, 10; 9) as a function of the joint movement or swivel.

2. Joint according to claim 1, characterised in that the location variation of at least one of said hinge points (7, 8) takes place on an orbital circuit about a rotary axis (13) provided on the hinge joint member (1; 9).

3. Joint according to claim 2, characterised in that the rotary axis (13) runs coaxial to a prominent axis (3; 21) of the basic mechanism of the joint.

4. Joint according to claim 1, characterised in that the location variation of the at least one hinge point (7, 8) is a sliding movement along a given slide path.

5. Joint according to claim 2 or 3, characterised in that the positive mechanical coupling is a one-stage spur gear (11, 12, 13; 11, 12, 21).

6. Joint according to claim 2 or 3, characterised in that the positive mechanical coupling is a planetary gear (15, 16, 17).

7. Joint according to claim 2 or 3, characterised in that the positive mechanical coupling is a differential gear.

8. Joint according to one of claims 1 to 4, characterised in that the positive mechanical coupling is a rod mechanism.

9. Joint according to claim 8, characterised in that the at least one hinge point (7) is the end member of a slider-crank mechanism (30; 31) with the inner or outer slider.

10. Joint according to claim 8, characterised in that the rod mechanism is a double-crank mechanism (18, 24) with coupling rod (19, 23).

11. Joint according to claims 1, 2 and 5 as a monocentric artificial knee joint, characterised in that the joint lower part (2) is non-rotatably connected with a first spur gear (11) arranged coaxial to the joint axis (3) and engaging with a second spur gear (12) which is pivoted with its rotary axis (13) parallel to the joint axis (3) in front of (stretching side) or behind (bending side) this at the joint upper part (1) and which has a cranking arm (14) on which the control element (4) is hinged via at least one hinge point (7).

12. Joint according to claim 11, characterised in that in the stretching position the joint axis (3) and rotary axis (13) lie in one plane perpendicular to the longitudinal axis of the orthopaedic aid.

13. Joint according to claims 1, 2 and 5 as a polycentric artificial knee joint in the form of a four-part kinematic link chain where the joint upper part (1) is connected to the joint lower part (2) via a front (stretching side) and a rear (bending side) guide rod (9, 10), characterised in that the front guide rod (9), coaxial to its upper joint axis (20) connecting it to the joint upper part (1), is non-rotatably connected with a first spur gear (11) engaging in a second spur gear (12) which is pivoted at the upper joint axis (21) connecting the rear guide rod (10) with the joint upper part (1) and has a crank arm (14) at which the control element (4) is hinged via at least one hinge point (7).

14. Joint according to claim 11, 12 or 13, characterised in that the ratio between the rolling or reference diameters of the two spur gears (11, 12) is selected such that for a joint bending angle of 90°, the action in the control element (4) is reversed.

15. Joint according to claim 13 or 14, characterised in that in the stretching position, the upper joint axis (20) of the front guide rod (9) lies above the upper joint axis (21) of the rear guide rod (10) and the lower joint axis (26) of the front guide rod (9) lies below the lower joint axis (25) of the rear guide rod (10).

16. Joint according to claim 13, 14 or 15, characterised in that the distance (1) between the two joint axes (21, 25) of the rear guide rod (10) corresponds to the distance (1) between the two joint axes (25, 26) of the joint lower part (2).

17. Joint according to any of claims 13 to 16, characterised in that the stretching position is defined by a stretching stop (32, 33, 34) acting directly between joint upper part (1) and joint lower part (2).

18. Joint according to claim 17, characterised in that the stretching stop (32, 33, 34) has two longitudinally movable stop buffers (32) arranged in parallel, which are contacted by an adjustment screw (34) via a common cross-piece (33).

19. Joint according to any of claims 13 to 18, characterised in that the joint lower part (2) has a releasable connection in the form of a fine thread (35) for connecting the parts of the prosthesis.

## Revendications

1. Articulation ou liaisons pivotantes sur prothèses et orthèses orthopédiques, comportant une partie supérieure d'articulation (1), une partie inférieure d'articulation (2) et, dans un mode de réalisation polycentrique, des bielles (9, 10) supplémentaires disposées entre ces parties d'articulation (la partie supérieure et la partie inférieure d'articulation ainsi que les bielles sont globalement désignées ci-dessous par "maillons"), et comportant un élément de commande (4) qui agit des deux côtés, chaque fois sur un maillon (11; 1, 2) (ci-dessous appelé "maillon d'articulation"), des deux côtés, chaque fois par l'intermédiaire d'un point d'articulation (7, 8) disposé à distance d'un axe d'articulation (3; 20, 21; 25, 26) de telle sorte que l'écart entre ces points d'articulation (7, 8) se modifie en fonction du déplacement ou du pivotement de l'articulation, dans lequel au moins un desdits points d'articulation (7, 8) modifie de lui-même, en fonction du déplacement ou du pivotement de l'articulation, sa position par rapport à son maillon d'articulation (1; 1, 2), par l'intermédiaire d'un accouplement de transmission forcée (11, 12, 13, 14; 15, 16, 17; 18, 24; 30; 31) avec au moins un autre maillon (2; 9, 10; 9).

2. Articulation selon la revendication 1, caractérisée en ce que le changement de position d'au moins l'un des points d'articulation (7, 8) cités s'effectue sur une trajectoire tracée autour d'un axe de rotation (13) prévu sur le maillon de l'articulation (1; 9).

3. Articulation selon la revendication 2, caractérisée en ce que l'axe d'articulation (13) s'étend coaxialement par rapport à un axe (3; 21) proéminent du mécanisme de base de l'articulation.

4. Articulation selon la revendication 1, caractérisée en ce que le changement de position du point d'articulation (7, 8) au moins présent est un déplacement de coulissement s'étendant le long d'une trajectoire de coulissement prédéterminée.

5. Articulation selon la revendication 2 ou 3, caractérisée en ce que le couplage forcé mécanique est une transmission à engrenages droits (11, 12, 13; 11, 12, 21) à un seul étage.

6. Articulation selon la revendication 2 ou 3, caractérisée en ce que le couplage forcé mécanique est une transmission à engrenages planétaires (15, 16, 17).

7. Articulation selon la revendication 2 ou 3, caractérisée en ce que le couplage forcé mécanique est une transmission à engrenages différentiels.

8. Articulation selon l'une des revendications 1 à 4, caractérisée en ce que le couplage forcé mécanique est une transmission à tiges.

9. Articulation selon la revendication 8, caractérisée en ce que le point d'articulation (7) au moins présent constitue le maillon final d'une transmission à bielle-manivelle (30; 31), comportant un embiellage intérieur ou extérieur

10. Articulation selon la revendication 8, caractérisée en ce que la transmission à tiges est une transmission à double manivelle (18, 24) comportant une tige de couplage (19, 23).

11. Articulation selon les revendications 1, 2 et 5, faisant office d'articulation monocentrique du genou pour prothèse, caractérisée en ce que la partie inférieure d'articulation (2) est assujettie en rotation à une première roue droite (11), disposée coaxialement par rapport à l'axe d'articulation (3), s'engrenant sur une deuxième roue droite (12), tourillonnant sur la partie supérieure d'articulation (1), avec son axe de rotation (13) orienté parallèlement par rapport à l'axe de rotation (3), devant (côté extension) ou derrière (côté flexion) l'axe de rotation (3) à la partie supérieure d'articulation (1), et présentant un bras de manivelle (14), auquel l'élément de commande (4) est articulé, par l'intermédiaire du au moins un point d'articulation (7).

12. Articulation selon la revendication 11, caractérisée en ce que, dans la position d'extension, l'axe d'articulation (3) et l'axe de rotation (13) sont situés dans un plan perpendiculaire par rapport à l'axe longitudinal du moyen auxiliaire orthopédique.

13. Articulation selon les revendications 1, 2 et 5, faisant office d'articulation polycentrique du genou pour prothèse, se présentant sous forme d'une chaîne d'articulation cinématique à quatre maillons, la partie supérieure d'articulation (1) étant reliée à la partie inférieure d'articulation (2), par l'intermédiaire d'une bielle avant (9) (côté extension) et d'une bielle arrière (côté flexion) (10), caractérisée en ce que la bielle avant (9) est assujettie en rotation, coaxialement par rapport à son axe d'articulation supérieur (20), la première roue droite (11) s'engrenant sur une deuxième roue droite (12), montée de façon à pouvoir tourillonner sur l'axe d'articulation supérieur (21), reliant la bielle arrière (10) à la partie d'articulation (1) et présentant un bras de manivelle (14), auquel l'élément de commande (4) est articulé par l'intermédiaire du au moins un point d'articulation (7).

14. Articulation selon la revendication 11, 12 ou 13, caractérisée en ce que le rapport entre le diamètre du cercle de roulement ou du cercle primitif des deux roues droites (11, 12) est choisi tel qu'en cas d'un angle de flexion d'articulation de 90°, il se produise une inversion de l'action dans l'élément de commande (4).

15. Articulation selon la revendication 13 ou 14, caractérisée en ce que, dans la position d'extension, l'axe d'articulation supérieur (20) de la bielle avant (9) est situé au-dessus de l'axe d'articulation supérieur (21) de la bielle arrière (10), et l'axe d'articulation inférieur (26) de la bielle avant (9) est situé au-dessous de l'axe d'articulation inférieur (25) de la bielle arrière (10).

16. Articulation selon la revendication 13, 14 ou 15, caractérisée en ce que la distance (l) entre les deux axes d'articulation (21, 25) de la bielle arrière (10) correspond à la distance (l) entre les deux axes d'articulation (25, 26) de la partie inférieure d'articulation (2).

17. Articulation selon l'une des revendications 13 à 16, caractérisée en ce que la force d'extension est définie par l'entrée en action d'une butée d'extension (32, 33, 34) agissant directement entre la partie supérieure d'articulation (1) et la partie inférieure d'articulation (2).

18. Articulation selon la revendication 17, caractérisée en ce que la butée d'extension (32, 33, 34) présente deux tampons de butée (32), disposés parallèlement et déplaçables longitudinalement, sollicités au moyen d'une vis de réglage (34), par l'intermédiaire d'une traverse (33) commune.

19. Articulation selon l'une des revendications 13 à 18, caractérisée en ce que la partie inférieure de l'articulation (2) présente une liaison désolidarisable, réalisée sous la forme d'un filetage fin (35), pour effectuer le raccordement de parties de prothèses.
